(19) **Europäisches Patentamt / European Patent Office / Office européen des brevets**

(11) **EP 4 236 885 B1**

(12) **FASCICULE DE BREVET EUROPEEN**

(45) Date de publication et mention de la délivrance du brevet:
**01.10.2025 Bulletin 2025/40**

(21) Numéro de dépôt: **21801902.4**

(22) Date de dépôt: **28.10.2021**

(51) Classification Internationale des Brevets (IPC):
***A61F 9/008*** (2006.01)

(52) Classification Coopérative des Brevets (CPC):
**A61F 9/00825; A61F 9/0084;** A61B 2018/20355;
A61F 2009/0087; A61F 2009/00872;
A61F 2009/00887; A61F 2009/00889;
A61F 2009/00897

(86) Numéro de dépôt international:
**PCT/EP2021/080018**

(87) Numéro de publication internationale:
**WO 2022/090408 (05.05.2022 Gazette 2022/18)**

(54) **SYSTEME DE DECOUPE D'UN TISSU OCULAIRE EN PORTIONS ELEMENTAIRES**

SYSTEM ZUM SCHNEIDEN VON AUGENGEWEBE IN ELEMENTARE TEILE

SYSTEM FOR CUTTING OCULAR TISSUE INTO ELEMENTARY PORTIONS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **29.10.2020 FR 2011099**

(43) Date de publication de la demande:
**06.09.2023 Bulletin 2023/36**

(73) Titulaire: **Keranova
42000 Saint Etienne (FR)**

(72) Inventeurs:
- **ROMANO, Fabrizio
01700 Beynost (FR)**
- **MAUCLAIR, Cyril
49440 Challain la Potherie (FR)**
- **BAUBEAU, Emmanuel
42000 Saint Etienne (FR)**

(74) Mandataire: **Be IP
Cabinet LTL SAS
Centre d'Entreprise et d'Innovation
56, Bd Niels Bohr
CS 52132
69603 Villeurbanne Cedex (FR)**

(56) Documents cités:
WO-A1-2016/055539    WO-A1-2018/020144
US-A1- 2015 164 689    US-A1- 2017 128 259
US-A1- 2019 314 194

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

## DOMAINE TECHNIQUE

La présente invention concerne le domaine technique des opérations chirurgicales réalisées au laser femtoseconde, et plus particulièrement celui de la chirurgie ophtalmologique, notamment pour des applications de découpes de cornées, ou de cristallins.

L'invention concerne un dispositif de découpe d'un tissu humain ou animal, telle qu'une cornée, ou un cristallin, au moyen d'une source laser femtoseconde.

Par source laser femtoseconde, on entend une source lumineuse, apte à émettre un faisceau laser sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

## ART ANTERIEUR

La source laser femtoseconde est un instrument apte à réaliser une découpe du tissu cornéen par exemple, en focalisant un faisceau laser dans le stroma de la cornée, et en réalisant une succession de petites bulles de gaz adjacentes.

Plus précisément, lors de la focalisation du faisceau laser dans la cornée, un plasma est généré par ionisation non-linéaire lorsque l'intensité du laser dépasse une valeur seuil, nommée seuil de claquage optique. Une bulle de gaz se forme alors, engendrant une disruption très localisée des tissus environnant. Ainsi, le volume réellement ablaté par le faisceau laser est très faible comparativement à la zone disruptée.

La zone découpée par le faisceau laser à chaque impulsion est très petite, de l'ordre du micron ou de la dizaine de microns selon la puissance et la focalisation du faisceau. Ainsi, une découpe lamellaire cornéenne ne peut être obtenue qu'en réalisant une série d'impacts contigus sur toute la surface de la zone à découper.

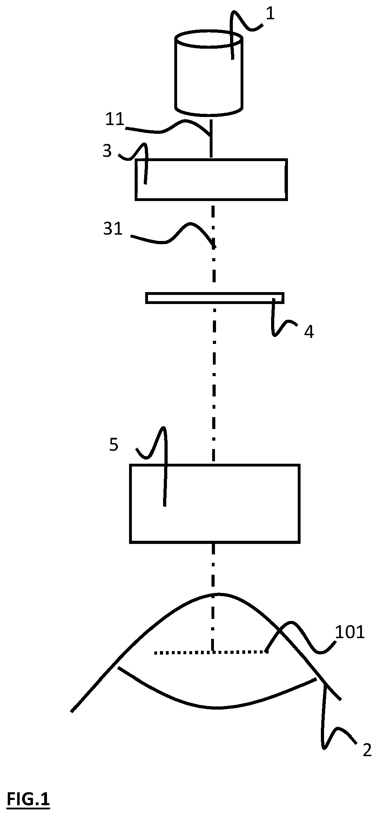

On connaît du document WO 2016/055539 un appareil de découpe d'un tissu oculaire 2 (humain ou animal) à partir d'une source laser femtoseconde 1. Cet appareil de découpe est illustré à la figure 1.

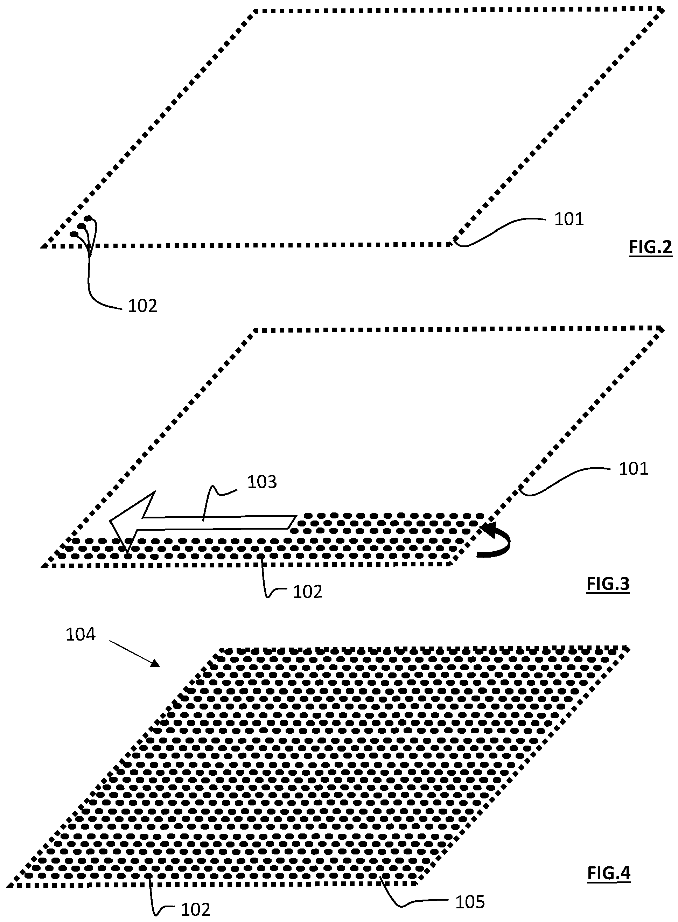

L'appareil de découpe permet, à partir d'un faisceau laser 11 issu d'une source laser femtoseconde 1, de générer une pluralité de points d'impact laser simultanés dans un plan focal 101 de l'appareil de découpe. Comme illustré à la figure 2, chaque point d'impact forme une bulle de gaz 102 respective. Pour générer simultanément une pluralité de points d'impact, l'appareil de découpe comprend un modulateur spatial de lumière 3 (connu sous le sigle SLM, de l'acronyme anglais *« Spatial Light Modulator »*). Un masque de phase est appliqué au SLM 3. Ce masque de phase permet de moduler la phase du front d'onde du faisceau laser 11 issu de la source laser femtoseconde 1. La modulation de phase du front d'onde permet de retarder ou d'avancer la phase des différents points de la surface du faisceau par rapport au front d'onde initial afin que chacun de ces points réalisent une interférence constructive en N points distincts dans le plan focal 101 de l'appareil de découpe. Cette redistribution d'énergie en une pluralité de points d'impact n'a lieu que dans un seul plan (i.e. le plan focal de l'appareil de découpe) et pas tout au long du chemin de propagation du faisceau laser modulé. Ainsi, la modulation de phase du front d'onde permet de générer un unique faisceau laser modulé 31 qui forme une pluralité de points d'impact seulement dans le plan focal 101 : le faisceau laser modulé 31 est unique tout au long de son chemin de propagation.

Pour découper un cristallin sur une surface de $1mm^2$, il faut réaliser environ 10000 points d'impact très proches les uns des autres. Le fait de générer simultanément plusieurs points d'impact permet de diminuer le temps nécessaire pour découper une surface de cristallin en augmentant la surface traitée avec un seul tir laser et en réduisant le nombre d'allers-retours nécessaires à réaliser plusieurs lignes de points adjacents.

La pluralité de points d'impact générés simultanément constitue un motif. En déplaçant 103 ce motif dans le plan focal 101 de l'appareil de découpe, il est possible de former un plan de découpe horizontal 104 comportant une multitude de bulles de gaz 102 (cf. figure 3). Pour déplacer le motif dans le plan focal 101, l'appareil de découpe comprend un dispositif de balayage 4, composé de miroirs galvanométriques pilotables, et/ou de platines permettant le déplacement d'éléments optiques, tels que des miroirs ou des lentilles. Ce dispositif de balayage 4 - positionné en aval du SLM 3 - permet de déplacer le faisceau laser modulé 31 suivant une trajectoire en va-et-vient le long d'une succession de segments constituant un chemin de déplacement du faisceau. On forme ainsi un plan de découpe horizontal 104 comportant une multitude de bulles de gaz 102 (cf. figure 4).

Lorsque la multitude de bulles de gaz 102 a été formée dans le plan focal 101 de l'appareil de découpe, la portion de cristallin située au-dessus du plan de découpe horizontal peut être désolidarisée de la portion de cristallin située au-dessous du plan de découpe horizontal en décrochant les ponts tissulaires 105 existants entre les bulles de gaz 102 à l'aide d'un outil.

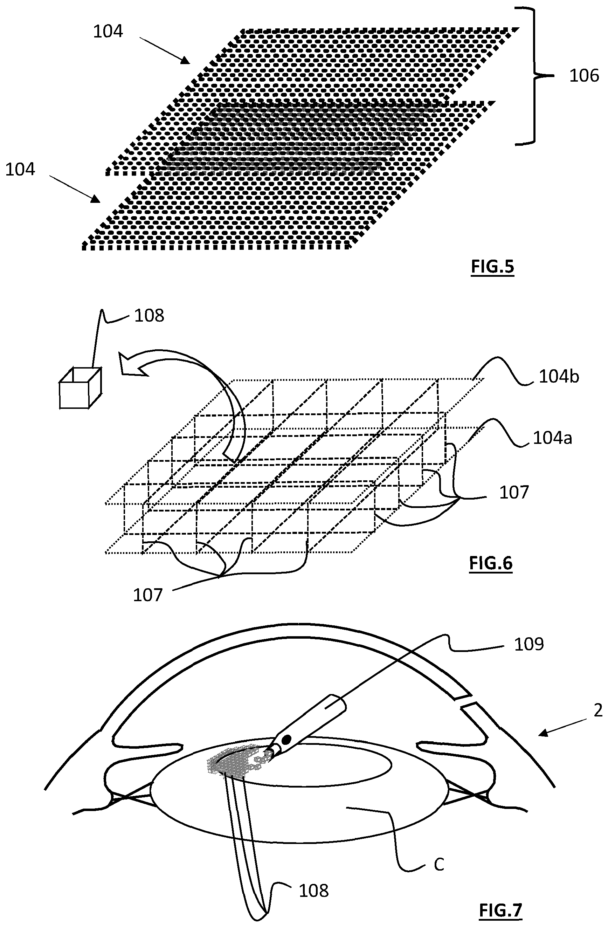

Lors d'une chirurgie de la cataracte, un empilement 106 de plans de découpe horizontaux 104 est formé en déplaçant le plan focal de l'appareil de découpe (cf. figure 5). Pour déplacer le plan focal 101, l'appareil de découpe comprend un dispositif optique de focalisation 5 - positionné en aval du dispositif de balayage 4 - composé notamment d'une (ou plusieurs) lentille(s) motorisée(s) pour permettre son (leur) déplacement en translation le long du chemin optique du faisceau laser modulé par le SLM 3 et dévié par le dispositif de balayage 4.

En déplaçant le plan focal 101 en différentes positions le long du chemin optique du faisceau laser, et en réitérant, pour chaque position du plan focal, les étapes :

- de génération d'un motif de points d'impact, et
- de déplacement du motif de points d'impact,

il est possible d'obtenir un empilement 106 de plan de découpe horizontaux 104. Les différentes tranches de cristallin définies par ces plans de découpe horizontaux 104 peuvent alors être séparées les unes des autres.

En plus des plans de découpe horizontaux 104, il est souhaitable de réaliser des plans de découpe verticaux 107 dans le cristallin. Ces plans verticaux 107 sont réalisés entre deux plans horizontaux successifs (réalisation d'un plan de découpe horizontal inférieur 104a puis réalisation des plans de découpe verticaux 107 puis réalisation d'un plan de découpe horizontal supérieur 104b). Ceci permet de subdiviser le cristallin C en cubes 108 pouvant être aspirés par une canule d'aspiration 109 lors d'une chirurgie de la cataracte par exemple (cf. figure 7) contrairement aux systèmes actuels qui nécessitent un phacoémulsificateur à ultrasons.

A l'heure actuelle, un plan de découpe vertical 107 est obtenu en réalisant des lignes de bulles de gaz superposées dans le cristallin C. Pour la réalisation d'un plan de découpe vertical, le faisceau laser issu de la source laser n'est pas modulé en phase. A chaque impulsion de la source laser femtoseconde, un point d'impact unique est formé. Ce point d'impact permet de produire une bulle de gaz. En déplaçant le faisceau laser à l'aide du dispositif de balayage, il est possible de déplacer le point d'impact dans le plan focal de l'appareil de découpe. Ceci permet de réaliser une succession de petites bulles de gaz adjacentes, qui forme ensuite une ligne de découpe dans le plan focal de l'appareil de découpe. En déplaçant le plan focal - à l'aide du dispositif de focalisation - en différentes positions le long du chemin optique du faisceau laser, il est possible de superposer les lignes de bulles de gaz afin d'obtenir un plan de découpe vertical.

Un tel plan de découpe vertical étant réalisé *« point par point »*, l'opération de formation des différents plans de découpe verticaux est lente. En effet, à l'heure actuelle, les points d'impact sont réalisés à une vitesse moyenne de 300.000 impacts/seconde. Pour découper *« point par point »* un cristallin sur une surface d'environ 65mm$^2$, en tenant compte des temps pendant lesquels le laser arrête la production des impulsions en bout de segment pour permettre aux miroirs de se positionner sur le segment suivant, il faut en moyenne 15 secondes.

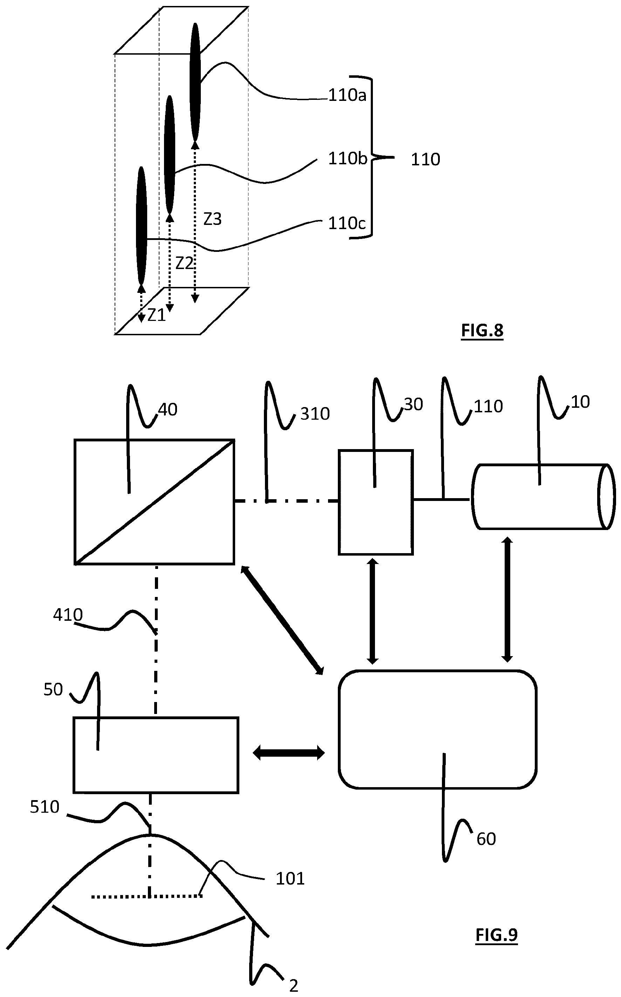

Pour remédier à cet inconvénient, et partant de l'appareil de découpe selon WO 2016/055539, les inventeurs ont essayé de réaliser des plans de découpe verticaux en mettant en œuvre le principe de démultiplication des points d'impact à partir de chaque impulsion de la source laser. En particulier, les inventeurs ont déterminé un masque de phase à appliquer au SLM pour générer plusieurs points d'impact 110 simultanés à des profondeurs Z1, Z2, Z3 différentes à partir d'un unique faisceau laser modulé (cf. figure 8). Par exemple, à partir d'un motif composé de trois (quatre, cinq, etc.) points d'impact 110a, 110b, 110c générés simultanément à des profondeurs différentes Z1, Z2, Z3, il est théoriquement possible, en déplaçant le motif le long d'un segment de déplacement grâce au dispositif de balayage, de générer simultanément trois (quatre, cinq, etc.) lignes de bulles de gaz superposées, ce qui diminue du facteur correspondant le temps nécessaire à la formation d'un plan de découpe vertical.

Toutefois, les inventeurs ont découvert que l'alignement des points d'impact 110 générés simultanément n'était pas suffisant, de sorte que les lignes de bulles de gaz n'étaient pas parfaitement superposées. Ce défaut d'alignement rend difficile le détachement des cubes de cristallin.

Le document WO 2018/020144 décrit un appareil de découpe de matériau diélectrique transparent ou semiconducteur. L'appareil comprend :

- une source laser générant un faisceau laser,
- un dispositif optique générateur de faisceau de Bessel configuré pour transformer une distribution spatiale d'intensité Gaussienne du faisceau laser en une distribution spatiale d'intensité de Bessel du faisceau laser transversalement à l'axe optique dans la zone de focalisation,
- un système optique passif comprenant un masque de phase et/ou d'amplitude configuré pour modifier la distribution spatiale de Bessel du faisceau laser transversalement et/ou longitudinalement par rapport à l'axe optique dans la zone.

Le document US 2015/164689 décrit un dispositif de découpe laser d'une matière transparente.

Le document US 2019/314194 décrit un système de capsulorhexis laser chirurgical comprenant :

- une source laser,
- un système de guidage de faisceau,
- un dispositif de focalisation de faisceau,
- un coupleur de faisceau configuré pour rediriger le faisceau de découpe laser focalisé, et
- une lentille d'interface patient.

Le document US 2017/128259 décrit un système de découpe pour la mise en œuvre d'une procédure de femto-fragmentation sur un tissu dans le cristallin d'un œil, qui requiert qu'un faisceau laser soit dirigé et focalisé vers un point focal dans le cristallin de l'œil.

Un but de la présente invention est de proposer une solution au problème de formation de plans de découpe verticaux dans un tissu oculaire (tel qu'une cornée ou un cristallin) à partir de l'appareil de découpe décrit dans WO 2016/055539.

## EXPOSE DE L'INVENTION

A cet effet l'invention propose un appareil de découpe d'un tissu humain ou animal, ledit appareil incluant une source laser femtoseconde configurée pour émettre un faisceau laser gaussien sous forme d'impulsions et un dispositif de traitement du faisceau laser gaussien, le dispositif de traitement étant disposé en aval de la source laser femtoseconde, le dispositif de traitement comprenant :

- un système de mise en forme positionné sur la trajectoire du faisceau laser gaussien, pour moduler la phase du front d'onde du faisceau laser gaussien, le système de mise en forme comprenant un modulateur spatial de lumière (SLM) et étant configuré pour produire un faisceau laser modulé à partir du faisceau laser gaussien,
- un scanner optique de balayage disposé en aval du système de mise en forme pour déplacer le faisceau laser modulé
- un système optique de focalisation en aval du système de mise en forme, pour focaliser le faisceau laser modulé dans un plan focal de l'appareil de découpe et pour déplacer le plan focal de l'appareil de découpe en une pluralité de positions le long d'un axe optique de propagation du faisceau laser modulé,

*remarquable en ce que* le dispositif de traitement comprend en outre une unité de commande pour piloter la source laser femtoseconde, le système de mise en forme, le scanner optique de balayage, et le système optique de focalisation, afin de réaliser au moins un plan de découpe vertical s'étendant parallèlement à l'axe optique, l'unité de commande étant configurée pour :

- appliquer au système de mise en forme, une consigne de modulation axiconique afin de produire un faisceau laser modulé de type Bessel à partir du faisceau laser gaussien, ladite consigne de modulation comportant un masque de phase (314, 315) émulant un axicon appliqué sur le modulateur spatial de lumière (SLM), ledit masque de phase (314, 315) ayant une symétrie de révolution autour d'un point central de symétrie, le niveau de gris de chaque point du masque de phase variant en fonction de la distance entre ledit point et le point central de symétrie, ledit faisceau laser modulé de type Bessel ayant un point d'impact permettant de générer une bulle de gaz oblongue dans le tissu et ainsi le découper sur une profondeur beaucoup plus grande qu'un faisceau gaussien,
- piloter le scanner optique de balayage pour déplacer le point d'impact du faisceau laser modulé de type Bessel le long d'un chemin optique déplacement pour former successivement une pluralité de bulle de gaz adjacentes, lesdites bulles de gaz constituant le plan de découpe vertical.

On entend, dans le cadre de la présente invention, par « *plan de découpe vertical* », un plan situé dans le tissu à traiter et s'étendant parallèlement à un axe optique de propagation du faisceau laser issu de l'appareil de découpe. On entend, dans le cadre de la présente invention, par « *plan de découpe horizontal* », un plan situé dans le tissu à traiter et s'étendant perpendiculairement à l'axe optique de propagation du faisceau laser issu de l'appareil de découpe.

On entend, dans le cadre de la présente invention, par « *point d'impact* » une zone du faisceau laser comprise dans son plan focal dans laquelle l'intensité dudit faisceau laser est suffisante pour générer une bulle de gaz dans un tissu.

On entend, dans le cadre de la présente invention, par « *points d'impact adjacents* », deux points d'impact disposés en regard l'un de l'autre et non séparés par un autre point d'impact.

On entend par « *points d'impact voisins* » deux points d'un groupe de points adjacents entre lesquels la distance est minimale.

On entend, dans le cadre de la présente invention, par « *motif* » une pluralité de points d'impact laser générés simultanément dans un plan de focalisation de l'appareil de découpe.

Ainsi, l'invention permet de modifier le profil d'intensité du faisceau laser dans le plan de découpe, d'une manière à pouvoir améliorer la qualité ou bien la vitesse de la découpe en fonction du profil choisi. Cette modification de profil d'intensité est obtenue par modulation de la phase du faisceau laser.

La modulation optique de phase est réalisée au moyen d'un masque de phase. L'énergie du faisceau laser incident est conservée après modulation, et la mise en forme du faisceau est réalisée en agissant sur son front d'onde. La phase d'une onde électromagnétique représente la situation instantanée de l'amplitude d'une onde électromagnétique. La phase dépend aussi bien du temps que de l'espace. Dans le cas de la mise en forme spatiale d'un faisceau laser, seules les variations dans l'espace de la phase sont considérées.

Le front d'onde est défini comme la surface des points d'un faisceau possédant une phase équivalente (i.e. la surface constituée des points dont les temps de parcours depuis la source ayant émis le faisceau sont égaux). La modification de la phase spatiale d'un faisceau passe donc par la modification de son front d'onde.

Cette technique permet de réaliser l'opération de découpe d'une manière plus rapide et plus efficace car elle met en œuvre plusieurs spots laser réalisant chacun une découpe et selon un profil contrôlé.

Dans le cadre de la présente invention, la modulation de phase du front d'onde permet de générer un unique faisceau laser modulé qui forme plusieurs points d'impact seulement dans le plan de découpe. Ainsi, le faisceau laser modulé est unique tout au long du chemin de propagation. La modulation de phase du front d'onde permet de retarder ou d'avancer la phase des différents points de la surface du faisceau par rapport au front d'onde initial afin que chacun de ces points réalisent une interférence constructive en N points distincts dans le plan focal d'une lentille. Cette redistribution d'énergie en une pluralité de points d'impact n'a lieu que dans un seul plan (i.e. le plan de focalisation) et pas tout au long du chemin de propagation du faisceau laser modulé.

Au contraire, le document US 2010/0133246 propose d'utiliser un système optique basé sur la phase et permettant de subdiviser un faisceau primaire en une pluralité de faisceaux secondaires ayant des angles de propagation différents.

La technique de modulation selon l'invention (par génération d'un unique faisceau laser modulé) permet de limiter les risques de dégradation de la qualité de la surface découpée. En effet, si une portion de l'unique faisceau laser modulé est perdue le long du chemin de propagation du faisceau, les intensités de tous les points d'impact du motif seront atténuées en même temps (conservation de l'homogénéité entre les différents points d'impact du motif) mais aucun point d'impact ne disparaitra dans le plan de découpe. Au contraire avec la technique de subdivision de faisceau proposée dans US 2010/0133246, si une portion de la pluralité de faisceaux secondaires est perdue le long du chemin de propagation, alors certains points d'impact du motif (correspondant aux points d'impact générés par les faisceaux secondaires perdus) seront absents dans le plan de découpe, ce qui dégrade sensiblement la qualité de la découpe effectuée.

Des aspects préférés mais non limitatifs de l'appareil de découpe sont les suivants :

- le plan focal objet du système de focalisation peut être positionné à une distance non nulle du plan focal image du système de mise en forme, de sorte que le point d'impact du faisceau laser modulé de type Bessel comporte :

  ○ un anneau focalisé dans le plan focal de l'appareil de découpe,
  ○ une ligne de concentration des rayons du faisceau laser modulé de type Bessel s'étendant en dehors du plan focal de l'appareil de découpe,

ladite ligne permettant de former la bulle de gaz oblongue, l'anneau ayant une intensité inférieure à l'intensité de la ligne ne permettant pas la formation de bulle de gaz ;

- l'unité de commande peut être programmée pour piloter le système optique de focalisation de sorte que le plan focal de l'appareil de découpe s'étende, selon l'axe optique, au-dessus de la position souhaitée pour le plan de découpe vertical ;
- l'unité de commande peut être programmée pour piloter le système optique de focalisation de sorte que le plan focal de l'appareil de découpe s'étende, selon l'axe optique, au-dessous de la position souhaitée pour le plan de découpe vertical ;

- l'unité de commande peut être en outre configurée pour piloter la source laser femtoseconde, le système de mise en forme, le scanner optique de balayage, et le système optique de focalisation, afin de réaliser au moins un plan de découpe horizontal s'étendant perpendiculairement à l'axe optique ;
- l'appareil de découpe peut être adapté pour réaliser successivement des plans de découpe horizontaux et verticaux de sorte à former des cubes de tissu :

  ○ l'unité de commande pilotant la source laser femtoseconde, le système de mise en forme, le scanner optique de balayage, et le système optique de focalisation pour réaliser un plan de découpe horizontal initial, puis
  ○ l'unité de commande pilotant la source laser femtoseconde, le système de mise en forme, le scanner optique de balayage, et le système optique de focalisation pour réaliser au moins un plan de découpe vertical situé au-dessus, selon l'axe optique, du plan de découpe horizontal initial, puis
  ○ l'unité de commande pilotant la source laser femtoseconde, le système de mise en forme, le scanner optique de balayage, et le système optique de focalisation pour réaliser un plan de découpe horizontal final au-dessus, selon l'axe optique, dudit et au moins un plan de découpe vertical ;

- pour la réalisation d'un plan de découpe horizontal, l'unité de commande peut être configurée pour :

  ○ appliquer un masque de phase multipoints au système de mise en forme pour produire un unique faisceau laser modulé multipoints le masque de phase multipoints étant calculé pour répartir l'énergie du faisceau laser modulé multipoints en au moins deux points d'impact dans le plan focal de l'appareil de découpe,
  ○ commander le déplacement du système de focalisation pour faire coïncider le plan focal de l'appareil de découpe à la profondeur souhaitée pour le plan de découpe horizontal,
  ○ activer la source laser femtoseconde, et
  ○ piloter le scanner optique de balayage pour déplacer les points d'impact de l'unique faisceau laser modulé multipoints le long d'un chemin de déplacement ;

- pour la réalisation d'un plan de découpe vertical, l'unité de commande (60) est configurée pour :

  ○ appliquer un masque de phase linéaire au système de mise en forme pour produire un faisceau laser modulé de Bessel,

◦ commander le déplacement du système de focalisation pour positionner le plan focal de l'appareil de découpe au-dessus ou au-dessous de la profondeur souhaitée pour le plan de découpe vertical,

◦ activer la source laser femtoseconde, et

◦ piloter le scanner optique de balayage pour déplacer le point d'impact faisceau laser modulé de Bessel le long d'un chemin de déplacement.

## BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :

- la figure 1 est une représentation schématique d'un appareil de découpe décrit dans WO 2016/055539 ;

- la figure 2 est une représentation schématique de bulles de gaz crées par des points d'impact dans un plan focal de l'appareil de découpe de la figure 1,

- la figure 3 est une représentation schématique de bulles de gaz crées successivement en déplaçant les points d'impact dans le plan focal de l'appareil de découpe de la figure 1,

- la figure 4 est une représentation schématique d'un plan de découpe horizontal obtenu grâce à l'appareil de découpe de la figure 1,

- la figure 5 est une représentation schématique d'un empilement de plans de découpe horizontaux obtenus grâce à l'appareil de découpe de la figure 1,

- la figure 6 est une représentation schématique de plans de découpe horizontaux et verticaux,

- la figure 7 est une représentation schématique d'un œil d'un patient,

- la figure 8 est une représentation schématique de points d'impact formés simultanément grâce à un SLM de l'appareil de découpe de la figure 1,

- la figure 9 est une représentation schématique d'un appareil de découpe selon l'invention,

- la figure 10a est une image d'un faisceau de type Bessel selon un profil longitudinal,

- la figure 10b est une image du faisceau de type Bessel selon un profil transverse,

- la figure 11 est une représentation schématique illustrant la focalisation d'un faisceau non diffractant de type Bessel,

- la figure 12a est une image d'un premier masque de phase permettant d'émuler le comportement d'un axicon négatif sur un SLM de l'appareil de découpe selon l'invention,

- la figure 12b est une image d'un deuxième masque de phase permettant d'émuler le comportement d'un axicon positif sur le SLM de l'appareil de découpe selon l'invention,

- la figure 13a est une représentation schématique d'un faisceau de type Bessel selon un profil longitudinal,

- la figure 13b est une représentation schématique du faisceau de type Bessel selon un profil transverse,

- la figure 14 est un schéma de montage partiel du dispositif de découpe,

- la figure 15 est une représentation schématique d'un faisceau de Bessel,

- la figure 16 est une représentation schématique illustrant la formation d'un plan de découpe vertical à partir d'un faisceau laser gaussien d'une part et d'un faisceau laser de Bessel d'autre part.

## EXPOSE DETAILLE DE L'INVENTION

L'invention concerne un système de découpe d'un tissu humain au moyen d'un laser femtoseconde. Dans la suite de la description, l'invention sera décrite, à titre d'exemple, pour la découpe d'un cristallin d'un œil humain ou animal.

### 1. _Appareil de découpe_

En référence à la figure 9, on a illustré un mode de réalisation de l'appareil de découpe selon l'invention. Celui-ci peut être disposé entre une source laser femtoseconde 10 et une cible à traiter 2.

La source laser femtoseconde 10 est apte à émettre un faisceau laser gaussien sous la forme d'impulsions. A titre d'exemple, la source laser femtoseconde 10 émet une lumière de 1030 nm de longueur d'onde, sous la forme d'impulsions de 400 femtosecondes. La source laser femtoseconde 10 possède une puissance de 20W et une fréquence de 500 kHz.

La cible 2 est par exemple un tissu humain ou animal à découper tel qu'une cornée ou un cristallin.

L'appareil de découpe comprend :

- un système de mise en forme 30 positionné sur la trajectoire du faisceau laser 110 issu du laser femtoseconde 10,

- un scanner optique de balayage 40 en aval du système de mise en forme 30,
- un système optique de focalisation 50 en aval du scanner optique de balayage 40, et
- une unité de commande 60.

Le système de mise en forme 30 permet de moduler la phase du faisceau laser 110 issu de la source laser femtoseconde 10. Ce système de mise en forme 30 est avantageusement un composant programmable.

Le scanner optique de balayage 40 permet d'orienter le faisceau laser modulé en phase 310 issu du système de mise en forme 30 pour déplacer le motif de découpe le long d'un chemin de déplacement prédéfini par l'utilisateur dans le plan focal 101 du système de découpe.

Le système optique de focalisation 50 permet de déplacer le plan focal 101 - correspondant au plan de découpe - du faisceau laser modulé et dévié 410.

L'unité de commande 60 permet de piloter le système de mise en forme 30, le scanner optique de balayage 40 et le système optique de focalisation 50.

Cet appareil de découpe est adapté pour former des plans de découpe horizontaux et verticaux. En fonction du type de plan de découpe souhaité (vertical ou horizontal), l'unité de commande 60 :

- configure le système de mise en forme pour moduler le faisceau laser 110 en fonction de l'aspect désiré pour les points d'impact, et
- contrôle le scanner optique de balayage 40 et du système optique de focalisation 50 pour générer le plan de découpe souhaité.

Comme il sera décrit plus en détails dans la suite, les inventeurs ont développé une solution de configuration originale de l'appareil de découpe pour la formation de plans de découpe verticaux.

## 2. Eléments de l'appareil de découpe

### 2.1. Système de mise en forme

Le système de mise en forme spatiale 30 du faisceau laser permet de faire varier la surface d'onde du faisceau laser 110 en fonction de la forme souhaitée pour le (ou les) point(s) d'impact du faisceau laser modulé.

Le système de mise en forme 30 comprend de préférence un modulateur spatial de lumière, connu sous le sigle SLM, de l'acronyme anglais *« Spatial Light Modulator ».*

Le SLM permet de moduler la répartition finale d'énergie du faisceau laser 110 issu de la source laser 10. Le SLM est un dispositif constitué d'une couche de cristaux liquides à orientation contrôlée permettant de façonner d'une manière dynamique le front d'onde, et donc la phase du faisceau laser 110. La couche de cristaux liquides d'un SLM est organisée comme une grille (ou matrice) de pixels. L'épaisseur optique de chaque pixel est contrôlée électriquement par orientation des molécules de cristal liquide appartenant à la surface correspondant au pixel. Le SLM exploite le principe d'anisotropie des cristaux liquides, c'est-à-dire la modification de l'indice des cristaux liquides, en fonction de leur orientation spatiale. L'orientation des cristaux liquides peut être réalisée à l'aide d'un champ électrique. Ainsi, la modification de l'indice des cristaux liquides modifie le front d'onde du faisceau laser.

D'une manière connue, le SLM met en œuvre un masque de phase, c'est-à-dire une carte déterminant comment la phase du faisceau laser 110 doit être modifiée pour obtenir une répartition d'amplitude donnée. Le masque de phase est une image bidimensionnelle dont chaque point est associé à un pixel respectif du SLM. Ce masque de phase permet de piloter l'indice de chaque cristal liquide du SLM en convertissant la valeur associée à chaque point du masque - représentée en niveaux de gris compris entre 0 et 255 (donc du noir au blanc) - en une valeur de commande - représentée en une phase comprise entre 0 et $2\pi$. Ainsi, le masque de phase est une consigne de modulation affichée sur le SLM pour entraîner en réflexion un déphasage spatial inégal du faisceau laser 110 illuminant le SLM. Bien entendu, l'homme du métier appréciera que la plage de niveau de gris peut varier en fonction du modèle de SLM utilisé. Par exemple dans certains cas, la plage de niveau de gris peut être comprise entre 0 et 220.

Différents masques de phase peuvent être appliqués au SLM en fonction du type de plan de découpe que souhaite réaliser l'utilisateur, à savoir :

- soit un plan de découpe vertical,
- soit un plan de découpe horizontal.

Pour la réalisation d'un plan de découpe vertical, le masque de phase utilisé (ci-après dénommé *« masque de phase linéaire »*) permet d'appliquer une modulation de phase linéaire avec symétrie de rotation. On obtient ainsi un faisceau laser modulé de type Bessel.

Pour la réalisation d'un plan de découpe horizontal, le masque de phase utilisé (ci-après dénommé *« masque de phase multipoints »*) permet d'appliquer une modulation de phase pour répartir l'énergie du faisceau laser en au moins deux points d'impact formant un motif dans le plan focal du système de découpe. On obtient ainsi un faisceau laser modulé de type multipoints.

### 2.1.1. Plan de découpe vertical

En ce qui concerne la découpe d'un plan vertical, les inventeurs proposent de moduler la phase du faisceau laser 110 issu de la source laser femtoseconde 10 de sorte à produire, en aval du système de mise en forme 30, un faisceau laser modulé 310 non diffractant de type Bessel.

Un faisceau de Bessel est dit *« non diffractant »* car il possède la propriété de garder un profil constant le long de l'axe optique de propagation du faisceau laser (ci-après dénommé *« axe optique »*), contrairement au comportement d'un faisceau laser Gaussien (tel que le faisceau laser 110 issu de la source laser femtoseconde 10) qui se disperse lorsqu'il est focalisé.

### 2.1.1.1. *Faisceau de Bessel*

Un faisceau de Bessel d'ordre zéro parfait peut être défini mathématiquement comme un faisceau dont le champ électrique (E) est formellement décrit par la fonction de Bessel d'ordre zéro de première espèce $J_0$ :

$$E(r, \phi, z)\ A_0 J_0(k_r r) e^{ik_z z}$$

où :

- $A_0$ est l'amplitude du champ électrique,
- $k_z$ et $k_r$ sont les vecteurs d'onde longitudinal et radial,
- $z$, $r$, et $\phi$ sont les composantes longitudinale, radiale, et azimutale.

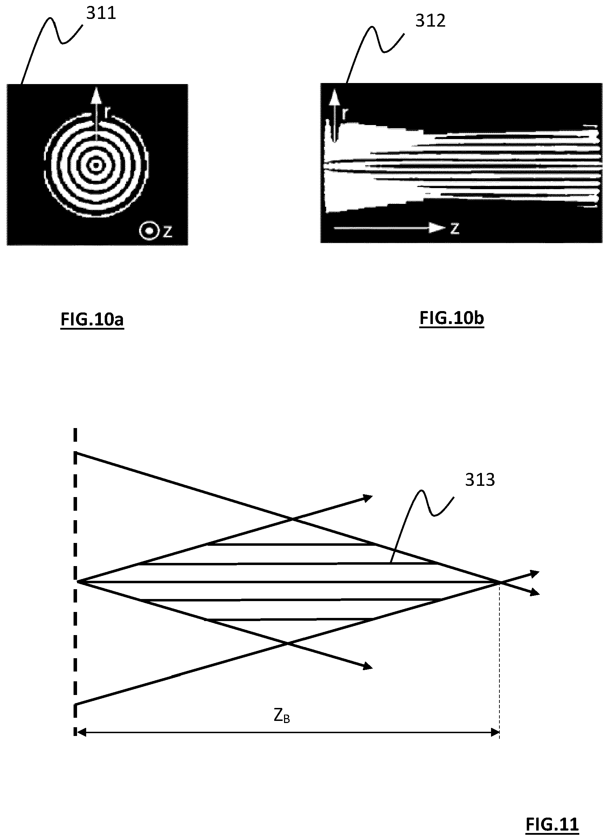

Le profil du faisceau de Bessel est représenté par un pic central d'intensité maximale entouré par des anneaux concentriques de plus faible intensité, tel qu'illustré aux figures 10a et 10b qui sont respectivement des vues de face et de côté d'un faisceau de Bessel relativement à son axe optique.

On observe sur les figures 10a, 10b une propagation avec un profil constant sur une distance de près de 100 $\mu$m (image 312, figure 10b) avec un diamètre de la tache de focalisation (image 311, figure 10a) inférieure à 1 $\mu$m. En comparaison, un faisceau gaussien présente généralement un profil de propagation constant sur 20 $\mu$m avec un diamètre de tache de focalisation de 1 $\mu$m.

En référence à la figure 11, la formation du faisceau de Bessel 313 résulte de l'interférence d'ondes planes dont les vecteurs d'onde forment une surface conique.

En théorie, l'extension transverse de la structure annulaire est infinie, ainsi que la distance de propagation non-diffractive.

En pratique, le faisceau de Bessel expérimental présente une distance de propagation non-diffractive finie $Z_B$ le long de l'axe optique du fait de la propagation finie observée en optique et de la quantité limitée d'énergie. Cette distance finie $Z_B$ de propagation non diffractive définit une zone de non-diffraction ZND.

Il est admis que $Z_B \gg Z_R$, $Z_R$ étant la distance de Rayleigh du faisceau gaussien usuel de taille transverse similaire. En d'autres termes, la profondeur (i.e. dimension selon une direction parallèle à l'axe optique de propagation du faisceau laser) de chaque point d'impact d'un faisceau de Bessel est beaucoup plus grande que la profondeur de chaque point d'impact avec un faisceau laser gaussien (tel que le faisceau laser issu de la source laser femtoseconde).

Ainsi, l'utilisation d'un faisceau de Bessel permet de découper une profondeur de tissu beaucoup plus importante qu'avec un faisceau Gaussien. En particulier, à partir d'un seul point d'impact d'un faisceau de Bessel, il est possible de découper un tissu sur une profondeur équivalente à celle de quatre points d'impact superposés d'un faisceau gaussien. Le déplacement, par le scanner optique de balayage, du point d'impact d'un faisceau de Bessel permet de générer un plan de découpe vertical parfaitement vertical quatre fois plus rapidement qu'avec un point d'impact de faisceau gaussien.

De par sa formation spécifique basée sur un front d'onde conique, le faisceau de Bessel présente des propriétés d'auto régénération remarquables, ce qui signifie que le faisceau peut se régénérer lui-même au sein de la zone de non-diffraction ZND après n'importe quel obstacle sur son chemin. Ceci permet d'assurer la qualité de la découpe des plans verticaux en garantissant la formation d'une bulle de gaz étendue à chaque tir de la source laser 10, même lorsqu'une partie du faisceau laser modulé 310 est masquée par un obstacle.

La génération d'une pluralité de points d'impact à différentes profondeurs à partir d'un faisceau laser modulé multipoints ne permet pas l'obtention d'un plan de découpe vertical de qualité équivalente à celle d'un plan de découpe vertical obtenu à partir d'un faisceau de Bessel. En effet, avec un faisceau laser modulé multipoints permettant la génération de plusieurs points d'impacts le long de l'axe optique, des imperfections dans la modulation de phase génèrent une lumière non contrôlée au niveau d'un plan focal du système optique de focalisation. Cette lumière non contrôlée interfère avec le motif de points d'impact souhaité. Il est donc impossible de contrôler précisément les intensités relatives des points d'impact dans le cas d'un faisceau laser modulé multipoints permettant la génération de plusieurs points d'impacts le long de l'axe optique.

Ainsi, du fait des capacités d'auto régénération du faisceau de Bessel, le point d'impact issu d'un faisceau de Bessel présente un avantage important par rapport aux points d'impacts simultanés formés le long de l'axe optique par un faisceau laser modulé multipoints.

### 2.1.1.2. *Masque de phase linéaire pour former un faisceau laser modulé de type Bessel*

Il existe différentes techniques pour générer un faisceau de Bessel à partir d'un faisceau laser gaussien. Ces techniques impliquent généralement une modulation de phase axiconique.

En particulier, le faisceau de Bessel peut être obtenu en utilisant une lentille conique connue sous le nom *« d'axicon »*. La lentille conique peut être concave/-creux (on parle *« d'axicon négatif »*) ou convexe/bombé (on parle *« d'axicon positif »*).

Les inventeurs proposent quant à eux d'utiliser le système de mise en forme 30 incluant le SLM pour générer le faisceau de Bessel afin d'éviter l'utilisation d'un élément optique/mécanique. A cet effet, un masque de phase linéaire (permettant d'émuler un axicon) est appliqué au SLM par l'unité de commande 60. Le SLM permet alors une modulation de phase conique du faisceau laser gaussien 110 issu de la source laser femtoseconde 10. Ainsi en utilisant le même SLM, il devient possible de réaliser un plan de découpe horizontal en multipoint, puis des plans de découpe verticaux en modalité faisceau de Bessel sans changer d'éléments optiques et donc en réduisant considérablement le temps de la procédure chirurgicale, compatible avec une application sur le globe oculaire du patient de moins de 3 minutes.

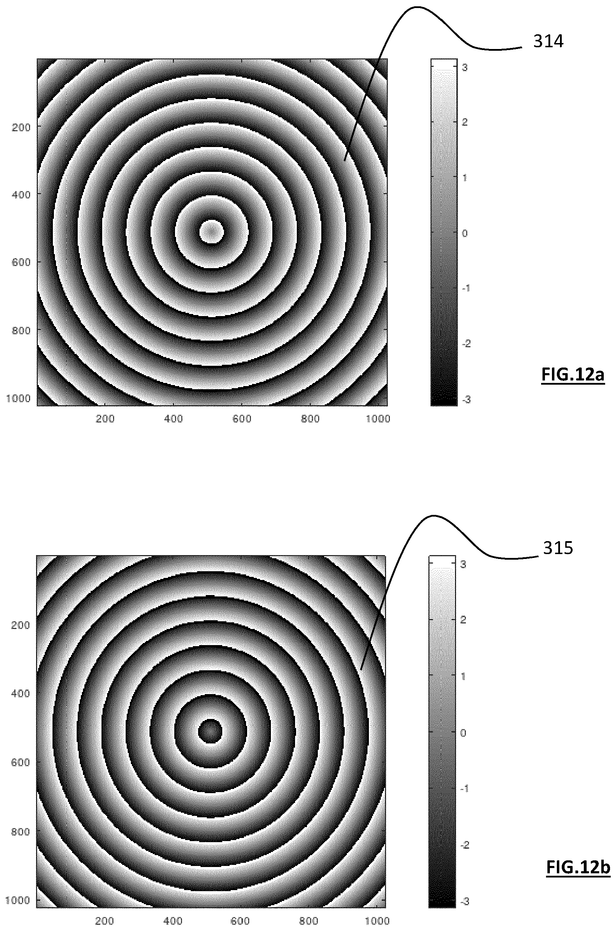

Deux exemples de tels masques de phase sont illustrés aux figures 12a et 12b. Lorsque l'un des premier et deuxième masques de phase est appliqué sur le SLM, le SLM est capable d'imprimer le profil de phase d'un axicon sur le faisceau laser gaussien 110 d'entrée pour obtenir un faisceau laser modulé de type Bessel 310 en sortie du système de mise en forme 30.

En référence à la figure 12a, le premier masque de phase linéaire (référencé 314) permet d'émuler le comportement d'un axicon négatif (i.e. axicon concave). En référence à la figure 12b, le deuxième masque de phase linéaire (référencé 315) permet d'émuler le comportement d'un axicon positif (i.e. axicon convexe). Ces premier et deuxième masques de phase présentent chacun une symétrie de révolution autour d'un point central de symétrie, le niveau de gris de chaque pixel variant en fonction de la distance entre ledit pixel et le point central de symétrie.

Lorsque l'un des masques de phase illustrés aux figures 12a et 12b est appliqué sur le SLM, le système de mise en forme 30 permet de former un faisceau laser modulé de type Bessel 310 (en sortie du système de mise en forme 30) à partir du faisceau laser gaussien 110 issu de la source laser femtoseconde 10 (en entrée du système de mise en forme 30). On obtient ainsi un faisceau laser modulé présentant une distribution spatiale d'intensité en faisceau de Bessel

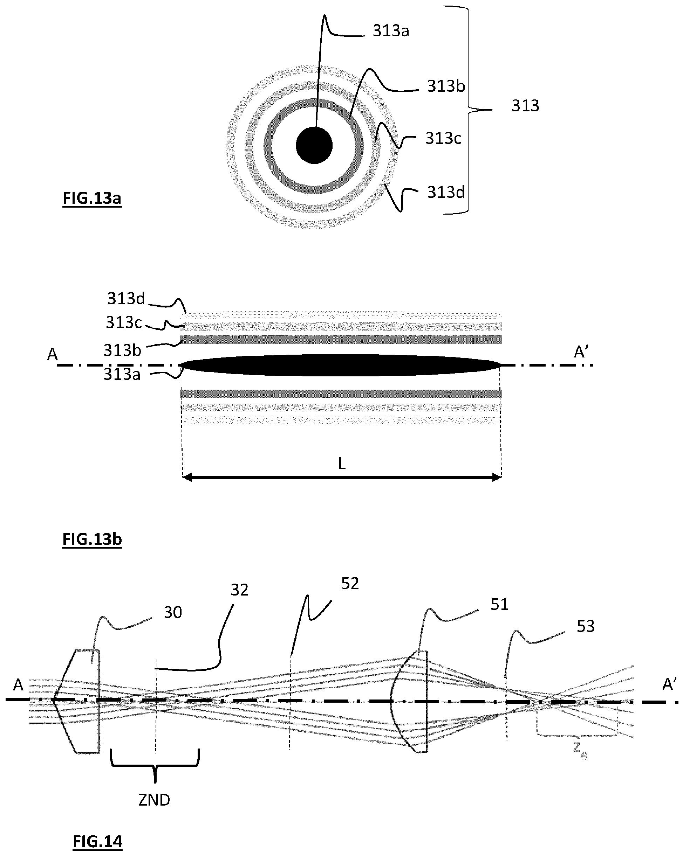

En référence aux figures 13a et 13b, ce faisceau laser modulé de type Bessel comprend, dans un plan transverse à l'axe optique :

- une tache centrale 313a d'intensité maximale, et
- plusieurs anneaux concentriques 313b, 313c, 313d d'intensité décroissante en fonction de la distance radiale à l'axe optique.

Ce faisceau de Bessel 313 s'étend sur une profondeur L le long de l'axe optique A-A' (i.e. dans la zone de non diffraction ZND du faisceau de Bessel). Le choix des valeurs de niveaux de gris des points du masque de phase linéaire permet d'optimiser la profondeur L du faisceau de Bessel 313 et donc le volume dans lequel son énergie est déposée.

Le masque de phase linéaire à appliquer au SLM du système de mise en forme pour former un faisceau laser modulé de Bessel peut être calculé :

- en utilisant un algorithme de partition (Vellekoop et Mosk, 2008),
- ou tout autre algorithme connu de l'homme du métier.

*2.1.1.3. Montage de l'appareil de découpe dans le cadre de la découpe d'un tissu à partir d'un faisceau laser modulé de type Bessel*

On a illustré à la figure 14 un schéma de montage de l'appareil de découpe. Ce schéma de montage est partiel en ce qu'il ne fait pas apparaître la source laser femtoseconde, et le scanner optique de balayage. Par ailleurs dans cette figure 14, le système optique de focalisation 50 (dans son ensemble) est représenté par une lentille équivalente 51, étant bien entendu pour l'homme du métier que le système optique de focalisation 50 ne consiste pas uniquement en une lentille fixe.

En référence à la figure 14, le faisceau de Bessel 313 est formé juste après le plan de modulation de phase conique, c'est-à-dire juste après le SLM du système de mise en forme 30. Le SLM simulant une lentille conique (axicon négatif ou positif), la tache centrale 313a d'intensité maximale du faisceau de Bessel se forme dans le plan focal image 32 du SLM.

La lentille équivalente 51 du système optique de focalisation 50 est disposé en aval du système de mise en forme 30, et est agencée de sorte que le plan focal objet 52 de la lentille équivalente s'étend à une distance non nulle du plan focal image 32 du système de mise en forme 30 long de l'axe optique.

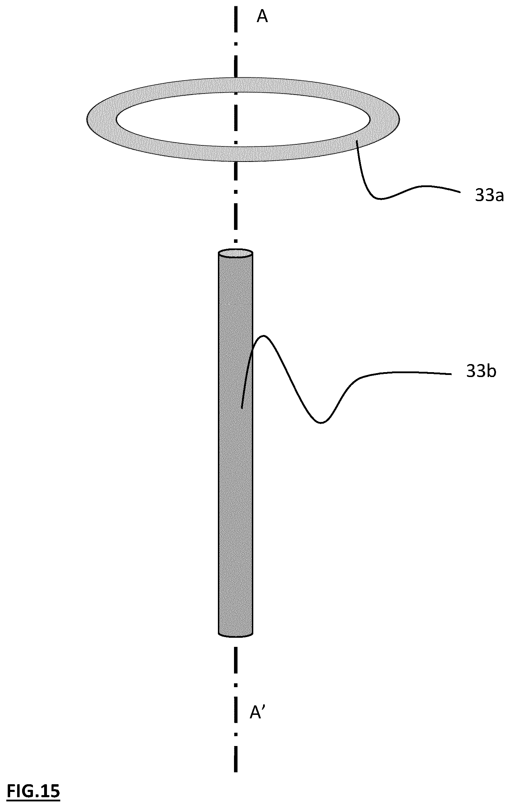

Ainsi, le plan focal objet 52 de la lentille équivalente 51 du système optique de focalisation 50 s'étend en dehors de la zone de non-diffraction ZND du faisceau de Bessel, de sorte qu'en sortie du système de découpe, un point d'impact tel qu'illustré à la figure 15 est obtenu. Ce point d'impact est composé :

- d'un anneau de Bessel 33a focalisé au plan focal image 53 de la lentille équivalente 51 (correspondant au plan focal de l'appareil de découpe),
- d'une ligne 33b de concentration des rayons du faisceau de Bessel - correspondant à l'image de la zone de non-diffraction ZND - ladite ligne 33b se formant en dehors du plan focal image 53 de la lentille équivalente 51.

Dans le cadre de la présente invention, c'est la ligne de concentration 33b du point d'impact qui est utilisée pour réaliser le plan de découpe vertical (l'énergie contenu dans l'anneau de Bessel n'est pas suffisante pour former une bulle de gaz).

La ligne 33b de concentration des rayons peut se former soit avant, soit après l'anneau 33a, en fonction du signe de la modulation de phase. En d'autres termes la position de la ligne 33b relativement à l'anneau 33a dépend du type d'axicon (positif ou négatif) émulé grâce au masque de phase linéaire.

Comme la zone de non-diffraction ZND de Bessel (i.e. la ligne 33b) est déplacée en dehors du plan focal du système de découpe, aucune interférence ne se produit avec la lumière non modulée. Ceci permet un meilleur contrôle du profile d'intensité sans pertes d'énergie lié au filtrage du faisceau.

### 2.1.2. *Plan de découpe horizontal*

En ce qui concerne la découpe d'un plan horizontal, les inventeurs proposent de moduler la phase du faisceau laser 110 issu de la source laser femtoseconde 10 de sorte à produire, en aval du système de mise en forme 30, un faisceau laser modulé de type multipoints.

A cet effet, un masque de phase multipoints à appliquer au SLM pour obtenir le faisceau laser modulé multipoints est calculé. Le masque de phase multipoints est généralement calculé par :

- un algorithme itératif basé sur la transformée de Fourier, tel qu'un algorithme de type *« IFTA »*, acronyme de l'expression anglo-saxonne *« Iterative Fourrier Transform Algorithm »,* ou par
- divers algorithmes d'optimisation, tels que des algorithmes génétiques, ou le recuit simulé.

Ce masque de phase multipoints est calculé pour former des pics d'intensité dans le plan focal de l'appareil de découpe, chaque pic d'intensité produisant un point d'impact respectif dans le plan focal de l'appareil de découpe.

Plus précisément, le masque de phase multipoints est calculé pour distribuer l'énergie du faisceau laser issu de la source laser en plusieurs points d'impact dans le plan focal de l'appareil de découpe. Cette modulation du front d'onde peut être vue comme un phénomène d'interférences en deux dimensions. Chaque portion du faisceau laser initial issu de la source est retardée ou avancée par rapport au front d'onde initial afin que chacune de ces portions soit redirigée de façon à réaliser une interférence constructive en N points distincts dans le plan focal d'une lentille. Cette redistribution d'énergie en une pluralité de points d'impact n'a lieu que dans un seul plan (i.e. le plan de focalisation) et pas tout au long du chemin de propagation du faisceau laser modulé. Ainsi, le faisceau laser multipoints obtenu (en sortie du système de mise en forme 30) est unique : l'observation du faisceau laser modulé avant ou après le plan focal de l'appareil de découpe (correspondant au plan focal du système optique de focalisation 50) ne permet pas d'identifier une redistribution de l'énergie en une pluralité de points d'impact distincts, du fait de ce phénomène qu'on peut assimiler à des interférences constructives (qui n'ont lieu que dans un plan et pas tout au long de la propagation comme dans le cas de la séparation d'un faisceau laser initial en une pluralité de faisceaux laser secondaires).

Le fait de disposer d'un unique faisceau laser modulé multipoint facilite l'intégration d'un système de balayage - tel qu'un scanner optique - pour déplacer la pluralité de points d'impact dans le plan focal. En effet, le diamètre d'entrée d'un système de balayage étant de l'ordre du diamètre du faisceau laser initiale issu de la source laser 10, l'utilisation d'un unique faisceau laser modulé multipoints (dont le diamètre est sensiblement égal au diamètre du faisceau laser initial) limite les risques d'aberration qui peuvent se produire avec la technique de subdivision de faisceau telle que décrite dans US 2010/0133246.

Le système de mise en forme 30 permet donc, à partir d'un faisceau laser gaussien générant un unique point d'impact, et au moyen du masque de phase multipoints appliqué au SLM, de répartir son énergie par modulation de phase de sorte à générer simultanément plusieurs points d'impact dans le plan focal de l'appareil de découpe, à partir d'un unique faisceau laser mis en forme par modulation de phase (un seul faisceau en amont et en aval du SLM). Ceci permet de diminuer le temps nécessaire pour réaliser un plan de découpe horizontal.

Par exemple dans le cas d'un faisceau laser modulé multipoints ayant trois points d'impact, le temps nécessaire à la réalisation d'un plan de découpe horizontal est réduit d'un facteur six (par rapport à la réalisation d'u même plan de découpe horizontal en utilisant un faisceau laser gaussien générant un unique point d'impact). L'homme du métier sait calculer une valeur en chaque point du masque de phase multipoints pour distribuer l'énergie du faisceau laser en différents points d'impact dans le plan focal de l'appareil de découpe.

### 2.2. *Scanner optique de balayage*

Le scanner optique de balayage 40 permet de dévier le faisceau laser modulé (de Bessel ou multipoints) 310 de sorte à déplacer le (ou les) point(s) d'impact en une pluralité de positions 43a-43c dans le plan de découpe.

Le scanner optique de balayage 4 comprend :

- un orifice d'entrée pour recevoir le faisceau laser modulé en phase 31 issu de l'unité de mise en forme 30,
- un (ou plusieurs) miroir(s) optique(s) pivotant autour d'au moins deux axes pour dévier le faisceau laser modulé en phase 310, et
- un orifice de sortie pour envoyer le faisceau laser modulé dévié 410 vers le système optique de focalisation 50.

Le scanner optique 4 utilisé est par exemple une tête de balayage IntelliScan III de la société SCANLAB AG.

Les orifices d'entrée et de sortie d'un tel scanner optique 40 présentent un diamètre de l'ordre de 10 à 20 millimètres, et les vitesses de balayage atteignables sont de l'ordre de 1m/s à 10m/s.

Le (ou les) miroir(s) est (sont) connecté(s) à un (ou des) moteur(s) pour permettre leur pivotement. Ce(s) moteur(s) pour le pivotement du (ou des) miroir(s) est (sont) avantageusement piloté(s) par l'unité de l'unité de commande 60 qui sera décrite plus en détails dans la suite.

L'unité de commande 60 est programmée pour piloter le scanner optique de balayage 40 de sorte à déplacer le (ou les) point(s) d'impact le long d'un chemin de déplacement contenu dans le plan de découpe.

Dans le cas d'un plan de découpe vertical, le chemin de déplacement comprend un segment. Dans ce cas, l'unité de commande 60 peut être configurée pour commander au scanner optique 40 un déplacement en va et vient du point d'impact de Bessel pour découper le plan de découpe sur toute sa profondeur. Par exemple, si le scanner optique 40 débute le segment par la gauche, il débutera ce segment par la droite au retour, puis par la gauche, puis par la droite et ainsi de suite sur toute la hauteur du plan de découpe.

Dans le cas d'un plan de découpe horizontal, le chemin de déplacement comprend une pluralité de segments de découpe. Le chemin de déplacement peut avantageusement présenter une forme de créneau.

Avantageusement, l'unité de commande 6 peut être programmée pour activer le laser femtoseconde 10 lorsque la vitesse de balayage du scanner optique 40 est supérieure à une valeur seuil. Ceci permet de synchroniser l'émission du faisceau laser 110 avec le balayage du scanner optique de balayage 40. Plus précisément, l'unité de commande 60 active le laser femtoseconde 10 lorsque la vitesse de pivotement du (ou des) miroir(s) du scanner optique 40 est constante. Ceci permet d'améliorer la qualité de découpe par la réalisation d'un surfaçage homogène du plan de découpe.

### 2.3. *Système optique de focalisation*

Le système optique de focalisation 50 permet de déplacer le plan focal de l'appareil de découpe en fonction du type de plan de découpe à réaliser.

Le système optique de focalisation 50 comprend :

- un orifice d'entrée pour recevoir le faisceau laser modulé en phase et dévié issu du scanner optique de balayage 40,
- une (ou plusieurs) lentille(s) motorisée(s) pour permettre son (leur) déplacement en translation le long du chemin optique du faisceau laser modulé et dévié, et

- un orifice de sortie pour envoyer le faisceau laser focalisé vers le tissu à traiter.

La (ou les) lentilles utilisées avec le système optique de focalisation 50 peuvent être des lentilles f-theta ou des lentilles télécentriques. Les lentilles f-theta et télécentriques permettent d'obtenir un plan de focalisation sur tout le champ XY, contrairement aux lentilles standard pour lesquelles il est courbe. Cela permet de garantir une taille de faisceau focalisé constante sur tout le champ. Pour les lentilles f-theta, la position du faisceau est directement proportionnelle à l'angle appliqué par le scanner tandis que le faisceau est toujours normal à l'échantillon pour les lentilles télécentriques.

L'unité de commande 60 est programmée pour piloter le déplacement de la (ou des) lentille(s) du système optique de focalisation 50 de sorte à déplacer le plan focal de l'appareil de découpe en fonction du type de plan de découpe à réaliser.

Dans le cas d'un plan de découpe horizontal, le plan de découpe correspond au plan focal de l'appareil de découpe. L'unité de commande 60 pilote le déplacement de la (ou des) lentille(s) du système optique de focalisation 50 pour focaliser le faisceau laser modulé et dévié 410 à une profondeur désirée correspondant à la profondeur du plan de découpe à réaliser.

Dans le cas d'un plan de découpe vertical, le plan de découpe peut être situé :

- au-dessous du plan focal de l'appareil de découpe dans le cas où le masque de phase linéaire utilisé permet au SLM d'émuler un axicon positif (l'anneau de Bessel 33a est situé au-dessus de la ligne de concentration 33b utilisée pour réaliser la découpe), dans ce cas l'unité de commande 60 pilote le système optique de focalisation 50 pour focaliser le faisceau laser modulé et dévié 410 à une profondeur désirée supérieure à la profondeur du plan de découpe à réaliser (afin que la ligne de concentration 33b du point d'impact soit localisée à la profondeur du plan de découpe à réaliser),

- au-dessus du plan focal de l'appareil de découpe dans le cas où le masque de phase linéaire utilisé permet au SLM d'émuler un axicon négatif (l'anneau de Bessel 33a est situé au-dessous de la ligne de concentration 33b utilisée pour réaliser la découpe) dans ce cas l'unité de commande 60 pilote le système optique de focalisation 50 pour focaliser le faisceau laser modulé et dévié 410 à une profondeur désirée inférieure à la profondeur du plan de découpe à réaliser (afin que la ligne de concentration 33b du point d'impact soit localisée à la profondeur du plan de découpe à réaliser).

Enfin, l'unité de commande 6 peut être programmée pour piloter le scanner optique de balayage 4 de sorte à faire varier l'aire découpée dans le plan de focalisation 21 entre deux plans de découpe successifs 22d, 22e. Ceci permet de faire varier la forme du volume 23 finalement découpé en fonction de l'application visée.

De préférence, la distance entre deux plans de découpe successifs est comprise entre 2 $\mu$m et 500 $\mu$m, et notamment :

- entre 2 et 20$\mu$m pour traiter un volume nécessitant une grande précision, par exemple en chirurgie réfractive, avec de préférence un espacement compris entre 5 et 10$\mu$m, ou
- entre 20 et 500$\mu$m pour traiter un volume ne nécessitant pas une grande précision, comme par exemple pour détruire la partie centrale d'un noyau cristallinien, avec de préférence un espacement compris entre 50 et 300$\mu$m.

Bien entendu, cette distance peut varier dans un volume 23 composé d'un empilement de plans de découpe 22a-22e.

## 2.4. *Unité de commande*

Comme indiqué précédemment, l'unité de commande 60 permet de contrôler les différents éléments constituant l'appareil de découpe, à savoir la source laser femtoseconde 10, le système de mise en forme 30, le scanner optique de balayage 40 et le système optique de focalisation 50.

L'unité de commande 60 est connectée à ces différents éléments par l'intermédiaire d'un (ou plusieurs) bus de communication permettant :

- la transmission de signaux de commande tels que

  - le signal d'activation à la source laser femtoseconde 10,
  - le masque de phase au système de mise en forme 30,
  - la vitesse de balayage au scanner optique de balayage 40,
  - la position du scanner optique de balayage 40 le long du chemin de déplacement,
  - la profondeur de découpe au système optique de focalisation 50.

- la réception de données de mesure issues des différents éléments du système tels que

  - la vitesse de balayage atteinte par le scanner optique, ou
  - la position du système optique de focalisation, etc.

L'unité de commande 60 peut être composée d'une (ou plusieurs) station(s) de travail, et/ou d'un (ou plusieurs) ordinateur(s) ou peut être de tout autre type connu de l'homme du métier. L'unité de commande 60 peut par exemple comprendre un téléphone portable, une tablette électronique (tel qu'un IPAD®), un assistant personnel (ou *« PDA »*, sigle de l'expression anglosaxonne *« Personal Digital Assistant »*), etc.

Dans tous les cas, l'unité de commande 60 comprend un processeur programmé pour permettre le pilotage de la source laser femtoseconde 10, du système de mise en forme 30, du scanner optique de balayage 40, du système optique de focalisation 50, etc.

Avantageusement, l'unité de commande 60 est programmée pour faire varier la forme du faisceau laser modulé entre deux plans de découpe successifs, notamment entre un plan de découpe horizontal et un plan de découpe vertical.

## 2.5. *Principe de fonctionnement*

On va maintenant décrire plus en détails le principe de fonctionnement de l'appareil de découpe en référence à la destruction d'un cristallin dans le cadre d'une opération de la cataracte.

Pour partitionner le cristallin en cubes susceptibles d'être aspirés par une canule d'aspiration, des plans de découpe horizontaux et verticaux sont formés en commençant par le plan de découpe horizontal le plus profond dans le cristallin et en empilant les plans de découpe verticaux et horizontaux successifs jusqu'au plan de découpe horizontal le plus superficiel dans le cristallin.

Dans une première étape, le plan de découpe horizontal le plus profond est réalisé. L'unité de commande 60 :

- applique un masque de phase multipoints au système de mise en forme 30 pour produire un faisceau laser modulé multipoints,
- commande le déplacement du système de focalisation 50 pour faire coïncider le plan focal de l'appareil de découpe avec le plan de découpe le plus profond souhaité,
- active la source laser femtoseconde 10, et
- pilote le déplacement du scanner optique de balayage le long du chemin optique (par exemple en créneau).

Une succession de tirs sont réalisés dans le plan focal de l'appareil de découpe. A chaque tir, plusieurs points d'impact focalisent simultanément dans le plan focal. Chaque point d'impact forme une bulle de gaz. Le scanner optique permet de déplacer les plusieurs points d'impacts dans le plan focal entre chaque tir. Lorsque toute la surface du plan de découpe horizontal est recouverte de bulles de gaz, le plan de découpe horizontal est finalisé.

Dans une deuxième étape, plusieurs plans de découpe verticaux adjacents sont ensuite réalisés avec l'appareil de découpe. Pour chaque plan de découpe vertical, l'unité de commande 60 :

- applique un masque de phase linéaire au système de mise en forme 30 pour produire un faisceau laser modulé de Bessel,
- commande le déplacement du système de focalisation 50 pour positionner la ligne de concentration 33b du point d'impact dans le plan de découpe (le plan de focalisation étant au-dessus ou en dessous du plan de découpe selon que l'axicon émulé sur le système de mise en forme soit un axicon positif ou négatif),
- active la source laser femtoseconde 10, et
- pilote le déplacement du scanner optique de balayage le long du chemin optique (par exemple un segment).

Une succession de tirs sont réalisés. A chaque tir, un point d'impact se forme, ce point d'impact incluant :

- un anneau 33a de faible intensité situé dans le plan focal de l'appareil de découpe,
- une ligne de concentration 33b de forte intensité située sur/sous le plan focal de l'appareil de découpe.

Chaque point d'impact forme une bulle de gaz oblongue selon l'axe optique de propagation du faisceau laser modulé. Le scanner optique permet de déplacer le point d'impact sous/sur le plan focal entre chaque tir. Lorsque tout le chemin de déplacement est recouvert de bulles de gaz, le plan de découpe vertical est finalisé.

Si la profondeur de la ligne 33b de concentration est inférieure à la profondeur souhaitée pour le plan de découpe vertical, alors l'unité de commande 60 peut contrôler le scanner optique de balayage 40 et le système optique de focalisation 50 pour déplacer en aller-retour le point d'impact le long du chemin optique en faisant varier la profondeur du plan focal de l'appareil de découpe entre l'aller et le retour.

On obtient ainsi plusieurs plans de découpe verticaux au-dessus du plan de découpe horizontal initial.

Dans une troisième étape, un plan horizontal supérieur est réalisé pour coiffer les plans de découpe verticaux. Ce plan de découpe horizontal est réalisé en selon la même méthode que celle décrite en référence à la première étape.

On obtient ainsi des cubes de cristallin définis entre les plans horizontaux et verticaux réalisés aux première deuxième et troisième étapes.

Celles-ci peuvent être réitérées pour réaliser un empilement de cubes de cristallin.

### 3. Conclusions

#### 3.1. Avantages associés à l'utilisation d'un faisceau de type Bessel

Comme indiqué précédemment, il est possible de découper une profondeur de tissu beaucoup plus importante avec un faisceau de Bessel, ce qui permet de générer un plan de découpe beaucoup plus rapidement qu'avec un faisceau Gaussien.

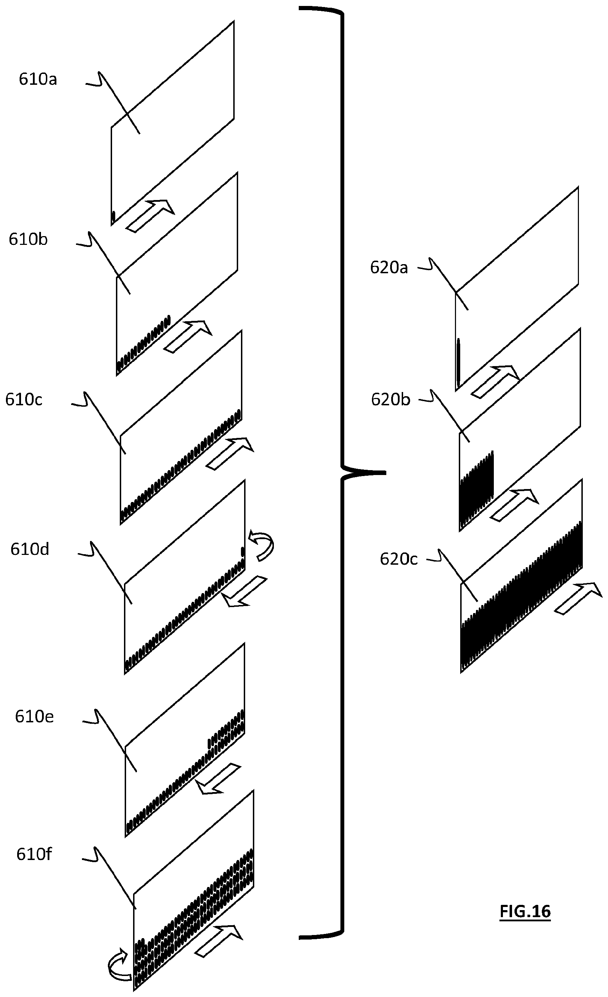

A titre indicatif, la figure 16 permet de comparer le temps nécessaire à la réalisation d'un plan de découpe vertical :

- à partir d'un faisceau gaussien d'une part (images 610a à 610f),
- à partir d'un faisceau de Bessel d'autre part (images 620a à 620c).

Dans le cas de l'utilisation d'un faisceau Gaussien générant un unique point d'impact déplacé par le scanner optique de balayage, il est nécessaire de réaliser quatre allers-retours pour former des bulles de gaz qui se superposent pour constituer le plan de découpe. Le temps nécessaire à la réalisation du plan de découpe vertical peut être formulé comme suit :

$$T1 = (8 \times t1) + (7 \times t2)$$

Où :

- T1 correspond au temp de découpe total
- t1 correspond au temps de parcours d'une ligne
- t2 correspond au temps de réalisation d'un demi-tour

En supposant $t1 \approx t2 = t$, alors le temps de découpe du plan est égal à 15 t dans le cas d'un faisceau Gaussien.

Dans le cas de l'utilisation d'un faisceau de Bessel, seul un aller-retour est nécessaire pour constituer le plan de découpe. Le temps nécessaire à la réalisation du plan de découpe vertical peut être formulé comme suit :

$$T2 = (2 \times t1) + (1 \times t2)$$

Où :

- T2 correspond au temp de découpe total
- t1 correspond au temps de parcours d'une ligne
- t2 correspond au temps de réalisation d'un demi-tour

En supposant $t1 \approx t2 = t$, alors le temps de découpe du plan est égal à 3 t dans le cas d'un faisceau de Bessel.

L'utilisation d'un SLM pour mettre en forme un faisceau Gaussien selon une consigne de modulation axiconique pour obtenir un faisceau laser module de Bessel générant un point d'impact oblong permet donc de diminuer d'un facteur cinq le temps nécessaire à la réalisation d'un plan de découpe vertical.

### 3.2. _Conclusion générale_

Ainsi, l'invention permet de disposer d'un outil de découpe tridimensionnelle efficace, contrairement aux outils actuels qui ne savent que réaliser des plans de découpe bidimensionnels (découpes verticales monospots en quartiers ou en bâtonnets sans possibilité de les combiner avec des découpes horizontales dans un temps acceptable).

En particulier, l'appareil de découpe est configuré pour réaliser une opération de découpe chirurgicale d'une manière rapide et efficace. Le SLM permet de façonner d'une manière dynamique le front d'onde du faisceau laser issu de la source laser femtoseconde puisqu'il est paramétrable numériquement :

- les plans de découpe horizontaux sont réalisés en utilisant un masque de phase multipoints,
- les plans de découpe verticaux sont réalisés en utilisant un masque de phase linéaire.

Le changement de masque de phase étant réalisé en quelques millisecondes, l'enchaînement des plans de découpe successivement horizontaux puis verticaux et ainsi de suite, se fait extrêmement rapidement sans avoir à mobiliser des éléments optiques/mécaniques, ce qui confère à cette invention son caractère unique permettant de découper un cristallin en 10.000 à 20.000 cubes en un temps de l'ordre de 30 secondes, alors qu'il faudrait entre 5 et 10 minutes aux systèmes actuels pour faire l'équivalent, ce qui est bien entendu inacceptable du point de vue du confort et de la sécurité du patient.

L'invention a été décrite pour des opérations de découpes d'un cristallin dans le domaine de la chirurgie ophtalmologique, mais il est évident qu'elle peut être utilisée pour d'autre type d'opération en chirurgie ophtalmologique sans sortir du cadre de l'invention. Par exemple, l'invention trouve une application dans la chirurgie réfractive cornéenne, tel que le traitement des amétropies, notamment myopie, hypermétropie, astigmatisme, dans le traitement de la perte d'accommodation, notamment la presbytie.

L'invention trouve également une application dans le traitement de la cataracte avec incision de la cornée, découpe de la capsule antérieure du cristallin, et fragmentation du cristallin. Enfin, d'une manière plus générale, l'invention concerne toutes les applications cliniques ou expérimentales sur la cornée ou le cristallin d'un œil humain ou animal.

D'une manière encore plus générale, l'invention concerne le domaine large de la chirurgie au laser et trouve une application avantageuse lorsqu'il s'agit de découper et plus particulièrement vaporiser des tissus mous humains ou animaux, à teneur en eau élevée.

Le lecteur aura compris que de nombreuses modifications peuvent être apportées à l'invention décrite précédemment sans sortir matériellement des nouveaux enseignements et des avantages décrits ici.

### Revendications

1.  Appareil de découpe d'un tissu humain ou animal, ledit appareil incluant une source laser femtoseconde (10) configurée pour émettre un faisceau laser gaussien sous forme d'impulsions et un dispositif de traitement du faisceau laser gaussien, le dispositif de traitement étant disposé en aval de la source laser femtoseconde (10), le dispositif de traitement comprenant :

    - un système de mise en forme (30) positionné sur la trajectoire du faisceau laser gaussien, pour moduler la phase du front d'onde du faisceau laser gaussien, le système de mise en forme (30) comprenant un modulateur spatial de lumière (SLM) et étant configuré pour produire un faisceau laser modulé à partir du faisceau laser gaussien,
    - un scanner optique de balayage (40) disposé en aval du système de mise en forme pour déplacer le faisceau laser modulé
    - un système optique de focalisation (50) en aval du système de mise en forme (30), pour focaliser le faisceau laser modulé dans un plan focal de l'appareil de découpe et pour déplacer le plan focal de l'appareil de découpe en une pluralité de positions le long d'un axe optique (A-A') de propagation du faisceau laser modulé, _caractérisé en ce que_ le dispositif de traitement comprend en outre une unité de commande (60) pour piloter la source laser femtoseconde (10), le système de mise en forme (30), le scanner optique de balayage (40), et le système optique de focalisation (50), afin de réaliser au moins un plan de découpe vertical s'étendant parallèlement à l'axe optique (A-A'), l'unité de commande (60) étant configurée pour :

        - appliquer au système de mise en forme (30), une consigne de modulation axiconique afin de produire un faisceau laser modulé de type Bessel à partir du faisceau laser gaussien, ladite consigne de modulation comportant un masque de phase (314, 315) émulant un axicon appliqué sur le modulateur spatial de lumière (SLM), ledit masque de phase (314, 315) ayant une symétrie de révolution autour d'un point central de symétrie, le niveau de gris de chaque point du masque de phase variant en fonction de la distance entre ledit point et le point central de symétrie, ledit faisceau laser modulé de type Bessel ayant un point d'impact permettant de générer une bulle de gaz oblongue dans le tissu et ainsi le découper sur une profondeur beaucoup plus grande qu'un faisceau gaussien,

- piloter le scanner optique de balayage pour déplacer le point d'impact du faisceau laser modulé de type Bessel le long d'un chemin optique déplacement pour former successivement une pluralité de bulle de gaz adjacentes, lesdites bulles de gaz constituant le plan de découpe vertical.

2. Appareil de découpe selon la revendication 1, *dans lequel* le plan focal objet du système de focalisation (50) est positionné à une distance non nulle du plan focal image du système de mise en forme (30), de sorte que le point d'impact du faisceau laser modulé de type Bessel comporte :

   ◦ un anneau (33a) focalisé dans le plan focal de l'appareil de découpe,
   ◦ une ligne (33b) de concentration des rayons du faisceau laser modulé de type Bessel s'étendant en dehors du plan focal de l'appareil de découpe,

   ladite ligne (33b) permettant de former la bulle de gaz oblongue, l'anneau (33a) ayant une intensité inférieure à l'intensité de la ligne (33b) ne permettant pas la formation de bulle de gaz.

3. Appareil de découpe selon l'une quelconque des revendications 1 ou 2, *dans lequel* l'unité de commande (60) est programmée pour piloter le système optique de focalisation (50) de sorte que le plan focal de l'appareil de découpe s'étende, selon l'axe optique (A-A'), au-dessus de la position souhaitée pour le plan de découpe vertical.

4. Appareil de découpe selon l'une quelconque des revendications 1 ou 2, *dans lequel* l'unité de commande (60) est programmée pour piloter le système optique de focalisation (50) de sorte que le plan focal de l'appareil de découpe s'étende, selon l'axe optique (A-A'), au-dessous de la position souhaitée pour le plan de découpe vertical.

5. Appareil de découpe selon l'une quelconque des revendications 1 à 4, *dans lequel* l'unité de commande (60) est en outre configurée pour piloter la source laser femtoseconde (10), le système de mise en forme (30), le scanner optique de balayage (40), et le système optique de focalisation (50), afin de réaliser au moins un plan de découpe horizontal s'étendant perpendiculairement à l'axe optique (A-A').

6. Appareil de découpe selon la revendication 5, *lequel* est adapté pour réaliser successivement des plans de découpe horizontaux et verticaux de sorte à former des cubes de tissu :

   - l'unité de commande pilotant la source laser femtoseconde (10), le système de mise en forme (30), le scanner optique de balayage (40), et le système optique de focalisation (50) pour réaliser un plan de découpe horizontal initial, puis
   - l'unité de commande pilotant la source laser femtoseconde (10), le système de mise en forme (30), le scanner optique de balayage (40), et le système optique de focalisation (50) pour réaliser au moins un plan de découpe vertical situé au-dessus, selon l'axe optique (A-A'), du plan de découpe horizontal initial, puis
   - l'unité de commande pilotant la source laser femtoseconde (10), le système de mise en forme (30), le scanner optique de balayage (40), et le système optique de focalisation (50) pour réaliser un plan de découpe horizontal final au-dessus, selon l'axe optique (A-A'), dudit et au moins un plan de découpe vertical.

7. Appareil de découpe selon la revendication 6, *dans lequel* pour la réalisation d'un plan de découpe horizontal, l'unité de commande (60) est configurée pour :

   - appliquer un masque de phase multipoints au système de mise en forme (30) pour produire un unique faisceau laser modulé multipoints le masque de phase multipoints étant calculé pour répartir l'énergie du faisceau laser modulé multipoints en au moins deux points d'impact dans le plan focal de l'appareil de découpe,
   - commander le déplacement du système de focalisation (50) pour faire coïncider le plan focal de l'appareil de découpe à la profondeur souhaitée pour le plan de découpe horizontal,
   - activer la source laser femtoseconde (10), et
   - piloter le scanner optique de balayage pour déplacer les points d'impact de l'unique faisceau laser modulé multipoints le long d'un chemin de déplacement.

8. Appareil de découpe selon l'une quelconque des revendications 6 ou 7, *dans lequel* pour la réalisation d'un plan de découpe vertical, l'unité de commande (60) est configurée pour :

   - appliquer un masque de phase linéaire au système de mise en forme (30) pour produire un faisceau laser modulé de Bessel,
   - commander le déplacement du système de focalisation (50) pour positionner le plan focal de l'appareil de découpe au-dessus ou au-dessous de la profondeur souhaitée pour le plan de découpe vertical,
- activer la source laser femtoseconde (10), et
- piloter le scanner optique de balayage pour déplacer le point d'impact faisceau laser modulé de Bessel le long d'un chemin de déplacement.

## Patentansprüche

1. Vorrichtung zum Schneiden eines menschlichen oder tierischen Gewebes, wobei die Vorrichtung eine Femtosekundenlaserquelle (10), die dazu ausgestaltet ist, einen Gaußschen Laserstrahl in der Form von Pulsen zu emittieren, und eine Einrichtung zur Verarbeitung des Gaußschen Laserstrahls umfasst, wobei die Verarbeitungseinrichtung nachgeschaltet zur Femtosekundenlaserquelle (10) angeordnet ist, wobei die Verarbeitungseinrichtung umfasst:

   - ein Formgebungssystem (30), das auf dem Weg des Gaußschen Laserstrahls positioniert ist, zum Modulieren der Phase der Wellenfront des Gaußschen Laserstrahls, wobei das Formgebungssystem (30) einen räumlichen Modulator für Licht (SLM) umfasst und dazu ausgestaltet ist, einen Laserstrahl zu erzeugen, der ausgehend von dem Gaußschen Laserstrahl moduliert wird,
   - einen optischen Abtastscanner (40), der nachgeschaltet zum Formgebungssystem angeordnet ist, um den modulierten Laserstrahl zu verlagern,
   - ein optisches Fokussierungssystem (50), das dem Formgebungssystem (30) nachgeschaltet ist, um den modulierten Laserstrahl in einer Brennebene der Schneidvorrichtung zu fokussieren und um die Brennebene der Schneidvorrichtung an einer Vielzahl von Positionen entlang einer optischen Ausbreitungsachse (A-A') des modulierten Laserstrahls zu verlagern, **dadurch gekennzeichnet, dass** die Verarbeitungseinrichtung ferner eine Steuereinheit (60) zum Steuern der Femtosekundenlaserquelle (10), des Formgebungssystems (30), des optischen Abtastscanners (40) und des optischen Fokussierungssystems (50) umfasst, um mindestens eine vertikale Schneidebene herzustellen, die sich parallel zur optischen Achse (A-A') erstreckt,
   wobei die Steuereinheit (60) ausgestaltet ist zum:

   - Anwenden, auf das Formgebungssystem (30), einer axikonischen Modulationsvorgabe, um einen modulierten Laserstrahl vom Typ Bessel ausgehend von dem Gaußschen Laserstrahl zu erzeugen, wobei die Modulationsvorgabe eine Phasenmaske (314, 315) umfasst, die ein Axikon emuliert, das auf den räumlichen Modulator für Licht (SLM) angewandt wird, wobei die Phasenmaske (314, 315) eine Drehsymmetrie um einen Symmetriemittelpunkt aufweist, der Graupegel jedes Punkts der Phasenmaske in Abhängigkeit von dem Abstand zwischen dem Punkt und dem Symmetriemittelpunkt variiert, der modulierte Laserstrahl vom Typ Bessel einen Auftreffpunkt aufweist, der das Erzeugen einer länglichen Gasblase in dem Gewebe und somit das Schneiden in einer viel größeren Tiefe als ein Gaußscher Strahl ermöglicht,
   - Steuern des optischen Abtastscanners, um den Auftreffpunkt des modulierten Laserstrahls vom Typ Bessel entlang eines optischen Verlagerungswegs zu verlagern, um aufeinanderfolgend eine Vielzahl von benachbarten Gasblasen zu bilden, wobei die Gasblasen die vertikale Schneidebene bilden.

2. Schneidvorrichtung nach Anspruch 1, wobei die Objektbrennebene des Fokussierungssystems (50) in einem Abstand von ungleich Null von der Bildbrennebene des Formgebungssystems (30) positioniert ist, derart dass der Auftreffpunkt des modulierten Laserstrahls vom Typ Bessel beinhaltet:

   ◦ einen Ring (33a), der in der Brennebene der Schneidvorrichtung fokussiert ist,
   ◦ eine Linie (33b) der Konzentration der Strahlen des modulierten Laserstrahls vom Typ Bessel, die sich außerhalb der Brennebene der Schneidvorrichtung erstreckt,

   wobei die Linie (33b) das Bilden der länglichen Gasblase ermöglicht, der Ring (33a) eine Intensität aufweist, die kleiner als die Intensität der Linie (33b) ist, die nicht die Gasblasenbildung ermöglicht.

3. Schneidvorrichtung nach einem der vorhergehenden Ansprüche 1 oder 2, wobei die Steuereinheit (60) dazu programmiert ist, das optische Fokussierungssystem (50) derart zu steuern, dass die Brennebene der Schneidvorrichtung sich entlang der optischen Achse (A-A') über der für die vertikale Schneidebene gewünschten Position erstreckt.

4. Schneidvorrichtung nach einem der Ansprüche 1 oder 2, wobei die Steuereinheit (60) dazu programmiert ist, das optische Fokussierungssystem (50) derart zu steuern, dass die Brennebene der Schneidvorrichtung sich entlang der optischen Achse (A-A') unter der für die vertikale Schneidebene gewünschten Position erstreckt.

**5.** Schneidvorrichtung nach einem der Ansprüche 1 bis 4, wobei die Steuereinheit (60) ferner dazu ausgestaltet ist, die Femtosekundenlaserquelle (10), das Formgebungssystem (30), den optischen Abtastscanner (40) und das optische Fokussierungssystem (50) zu steuern, um mindestens eine horizontale Schneidebene herzustellen, die sich senkrecht zur optischen Achse (A-A') erstreckt.

**6.** Schneidvorrichtung nach Anspruch 5, die dazu ausgebildet ist, aufeinanderfolgend horizontale und vertikale Schneidebenen herzustellen, derart dass Gewebewürfel gebildet werden:

- wobei die Steuereinheit die Femtosekundenlaserquelle (10), das Formgebungssystem (30), den optischen Abtastscanner (40) und das optische Fokussierungssystem (50) steuert, um eine horizontale Anfangsschneidebene herzustellen, dann
- die Steuereinheit die Femtosekundenlaserquelle (10), das Formgebungssystem (30), den optischen Abtastscanner (40) und das optische Fokussierungssystem (50) steuert, um mindestens eine vertikale Schneidebene herzustellen, die sich entlang der optischen Achse (A-A') über der horizontalen Anfangsschneidebene befindet, dann
- die Steuereinheit die Femtosekundenlaserquelle (10), das Formgebungssystem (30), den optischen Abtastscanner (40) und das optische Fokussierungssystem (50) steuert, um eine horizontale Endschneidebene herzustellen, die sich entlang der optischen Achse (A-A') über der mindestens einen vertikalen Schneidebene befindet.

**7.** Schneidvorrichtung nach Anspruch 6, wobei für die Herstellung einer horizontalen Schneidebene die Steuereinheit (60) ausgestaltet ist zum:

- Anwenden einer Mehrpunkt-Phasenmaske auf das Formgebungssystem (30), um einen einzelnen modulierten Mehrpunkt-Laserstrahl herzustellen, wobei die Mehrpunkt-Phasenmaske berechnet wird, um die Energie des modulierten Mehrpunkt-Laserstrahls auf mindestens zwei Auftreffpunkte in der Brennebene der Schneidvorrichtung zu verteilen,
- Steuern der Verlagerung des Fokussierungssystems (50), um zu bewirken, dass die Brennebene der Schneidvorrichtung mit der für die horizontale Schneidebene gewünschten Tiefe zusammenfällt,
- Aktivieren der Femtosekundenlaserquelle (10), und
- Steuern des optischen Abtastscanners, um die Auftreffpunkte des einzelnen modulierten Mehrpunkt-Laserstrahls entlang eines Verlagerungswegs zu verlagern.

**8.** Schneidvorrichtung nach einem der Ansprüche 6 oder 7, wobei für die Herstellung einer vertikalen Schneidebene die Steuereinheit (60) ausgestaltet ist zum:

- Anwenden einer linearen Phasenmaske auf das Formgebungssystem (30), um einen modulierten Bessel-Laserstrahl zu erzeugen,
- Steuern der Verlagerung des Fokussierungssystems (50), um die Brennebene der Schneidvorrichtung über oder unter der für die vertikale Schneidebene gewünschten Tiefe zu positionieren,
- Aktivieren der Femtosekundenlaserquelle (10), und
- Steuern des optischen Abtastscanners, um den Auftreffpunkt des modulierten Bessel-Laserstrahls entlang eines Verlagerungswegs zu verlagern.

**Claims**

**1.** A cutting apparatus for a human or animal tissue, said apparatus including a femtosecond laser source (10) configured to emit a Gaussian laser beam in the form of pulses, and a processing device of the Gaussian laser beam, the processing device being arranged downstream of the femtosecond laser source (10), the processing device comprising:

- a shaping system (30) positioned on the trajectory of the Gaussian laser beam, in order to modulate the phase of the wavefront of the Gaussian laser beam, the shaping system (30) comprising a spatial light modulator (SLM) and being configured to produce a modulated laser beam from the Gaussian laser beam,
- a sweeping optical scanner (40) arranged downstream of the shaping system in order to move the modulated laser beam,
- an optical focusing system (50) downstream of the shaping system (30), for focusing the modulated laser beam in a focal plane of the cutting apparatus and for moving the focal plane of the cutting apparatus into a plurality of positions along an optical axis (A-A') of propagation of the modulated laser beam,
*characterised in that* the processing device further comprises a control unit (60) for driving the femtosecond laser source (10), the shaping system (30), the sweeping optical scanner (40) and the optical focusing system (50), in order to produce at least one vertical cutting plane extending parallel to the optical axis (A-A'),

the control unit (60) being configured to:

- apply, to the shaping system (30), an axiconic modulation instruction in order to produce a Bessel-type modulated laser beam from the Gaussian laser beam, said modulation instruction including a phase mask (314, 315) emulating an axicon applied on the spatial light modulator (SLM), said phase mask (314, 315) having a rotational symmetry about a central symmetry point, the grey level of each point of the phase mask varying according to the distance between said point and the central symmetry point, said Bessel-type modulated laser beam having an impact point enabling an oblong gas bubble to be generated in the tissue and thus cutting it to a depth much greater than with a Gaussian beam,
- drive the sweeping optical scanner in order to move the impact point of the Bessel-type modulated laser beam along an optical movement path in order to successively form a plurality of adjacent gas bubbles, said gas bubbles constituting the vertical cutting plane.

2. The cutting apparatus according to claim 1, *wherein* the object focal plane of the focusing system (50) is positioned at a non-zero distance from the image focal plane of the shaping system (30), such that the impact point of the Bessel-type modulated laser beam includes:

○ a ring (33a) focused in the focal plane of the cutting apparatus,
○ a line (33b) of concentration of the rays of the Bessel-type modulated laser beam extending outside the focal plane of the cutting apparatus,

said line (33b) making it possible to form the oblong gas bubble, the ring (33a) having an intensity less than the intensity of the line (33b), not allowing gas bubble formation.

3. The cutting apparatus according to any one of claims 1 or 2, *wherein* the control unit (60) is programmed to drive the optical focusing system (50) such that the focal plane of the cutting apparatus extends, along the optical axis (A-A'), above the desired position for the vertical cutting plane.

4. The cutting apparatus according to any one of claims 1 or 2, *wherein* the control unit (60) is programmed to drive the optical focusing system (50) such that the focal plane of the cutting apparatus extends, along the optical axis (A-A'), below the desired position for the vertical cutting plane.

5. The cutting apparatus according to any one of claims 1 to 4, *wherein* the control unit (60) is further configured to drive the femtosecond laser source (10), the shaping system (30), the sweeping optical scanner (40), and the optical focusing system (50), in order to produce at least one horizontal cutting plane extending perpendicular to the optical axis (A-A').

6. The cutting apparatus according to claim 5, *which* is suitable for successively producing horizontal and vertical cutting planes so as to form cubes of tissue:

- the control unit driving the femtosecond laser source (10), the shaping system (30), the sweeping optical scanner (40) and the optical focusing system (50), in order to produce an initial horizontal cutting plane, then
- the control unit driving the femtosecond laser source (10), the shaping system (30), the sweeping optical scanner (40) and the optical focusing system (50), in order to produce at least one vertical cutting plane located above, along the optical axis (A-A'), the initial horizontal cutting plane, then
- the control unit driving the femtosecond laser source (10), the shaping system (30), the sweeping optical scanner (40) and the optical focusing system (50), in order to produce a final horizontal cutting plane above, along the optical axis (A-A'), said and at least one vertical cutting plane.

7. The cutting apparatus according to claim 6, *wherein* in order to produce a horizontal cutting plane, the control unit (60) is configured to:

- apply a multipoint phase mask to the shaping system (30) in order to produce a single multipoint modulated laser beam, the multipoint phase mask being calculated to distribute the energy of the multipoint modulated laser beam into at least two impact points in the focal plane of the cutting apparatus,
- control the movement of the focusing system (50) in order to make the focal plane of the cutting apparatus coincide with the desired depth for the horizontal cutting plane,
- activate the femtosecond laser source (10), and
- drive the sweeping optical scanner in order to move the impact points of the single multipoint modulated laser beam along a movement path.

8. The cutting apparatus according to any one of claims 6 or 7, *wherein* in order to produce a vertical cutting plane, the control unit (60) is configured to:

- apply a linear phase mask to the shaping system (30) in order to produce a Bessel modulated laser beam,
- control the movement of the focusing system (50) in order to position the focal plane of the cutting apparatus above or below the desired depth for the vertical cutting plane,
- activate the femtosecond laser source (10), and
- drive the sweeping optical scanner in order to move the impact point of the Bessel modulated laser beam along a movement path.

- apply a linear phase mask to the shaping system (30) in order to produce a Bessel modulated laser beam,

**FIG.1**

101

102

**FIG.2**

103

101

102

**FIG.3**

104

102

105

**FIG.4**

**FIG.5**

**FIG.6**

**FIG.7**

**FIG.8**

**FIG.9**

**FIG.10a**

**FIG.10b**

**FIG.11**

314

**FIG.12a**

315

**FIG.12b**

313a

313b

313

313c

313d

**FIG.13a**

313d

313c

313b

A ———————————————————————————————————————————— A'

313a

L

**FIG.13b**

32          52

30                          51

53

A ———————————————————————————————————————————— A'

$Z_B$

ZND

**FIG.14**

A

A'

33a

33b

**FIG.15**

610a

610b

610c

610d

610e

610f

620a

620b

620c

**FIG.16**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2016055539 A
- WO 2018020144 A
- US 2015164689 A
- US 2019314194 A
- US 2017128259 A
- US 20100133246 A